# EUROPEAN PATENT APPLICATION

(11) **EP 2 189 453 A1**
(43) Date of publication of application: **26.05.2010**
(21) Application number: 08305835.4
(22) Date of filing: 25.11.2008
(51) Int. Cl.: C07D 307/93, A61K 31/343, A61P 9/00

(54) **Rocaglaol derivatives as cardioprotectant agents**

(71) Applicant: Université Louis Pasteur, 67000 Strasbourg (FR)
(72) Inventor: DESAUBRY, Laurent, 67400, ILLKIRCH (FR); DESAUBRY, Gunsel Canan, 67400, ILLKIRCH (FR); CRESTEIL, Thierry, 92290, CHATENAY MALABRY (FR); TÜRKERI, Gülen, 67400, ILLKIRCH (FR)
(74) Representative: Gallois, Valérie

(57) **Abstract**

The present invention discloses new rocaglaol derivatives and the use of rocaglaol derivatives to prevent or to limit the cardiotoxicity of an antineoplastic agent, in particular to prevent or to limit the apoptosis of cardiomyocytes induced by such agent.

## Description

### Field of the Invention

The present invention relates to the prevention of antineoplastic agent cardiotoxicity by using new cardioprotectant agents.

### Background of the Invention

Cardiotoxicity is a major side effect of various antineoplastic agents including, for instance, anthracyclines, cyclophosphamide, fluorouracil, rituximab, cisplatine, gleevec or herceptin (Pai et al., 2000). Among them, anthracyclines are a class of chemotherapeutic agents which are used to treat a wide range of cancers, including, for instance, leukemias, lymphomas, and breast, uterine, ovarian, and lung cancers. Nevertheless, the clinical utility of anthracycline anticancer agents, especially doxorubicin, is limited by a progressive toxic cardiomyopathy linked to mitochondrial damage and cardiomyocyte apoptosis (Swain et al., 2003). This cardiotoxicity often presents as electrocardiogram changes and arrhythmias, or as a cardiomyopathy leading to congestive heart failure, sometime many years after treatment (Steinherz et al., 1995), and is related to a patient's cumulative lifetime dose. Some recent studies have revealed that when the cumulative dose of doxorubicin reaches 700 mg/m², the risks of developing congestive heart failure dramatically increase to 48% (Swain et al., 2003). Then, this dose is calculated during treatment, and anthracycline treatment is usually stopped or re-evaluated upon reaching the maximum cumulative dose of the particular anthracycline.
Important researches have been carried out to identify methods or drugs which are able to prevent or limit the cardiotoxicity of anthracyclines (Wounters et al., 2005). Several strategies have been explored (e.g. US 2007-834799, US 2007-711490, WO 2006/063091 or WO 2007/101925), but the use of dexrazoxane (Zinecard^{®}, Cardioxane^{®}) remains the only one with a clinical proven efficacy. Dexrazoxane is then usually used in conjunction with doxorubicin as a cardioprotectant agent to reduce the risk of cardiotoxicity side effects of anthracycline chemotherapy. As a derivative of EDTA, dexrazoxane chelates iron, but the precise mechanism by which it protects the heart is not known.
Nevertheless, the use of dexrazoxane does not provide a full protection against heart damages and some studies revealed that patients treated with it might have a lower anti-tumour response rate to the anthracycline treatment (Van Dalen, et al., 2005). Moreover, the effectiveness of dexrazoxane in children is not established yet.

Therefore, there is a strong need for cardioprotectant agents that can efficiently prevent the cardiotoxicity of anthracyclines or other antineoplastic agents without having any deleterious effect on the anti-tumour response. This need is still stronger for children who are more susceptible to cardiotoxicity of these agents.

### Summary of the Invention

The object of the present invention is to provide new cardioprotectant agents. The inventors have shown that rocaglaol and some of its derivatives are able to prevent or limit apoptosis induced by the cardiotoxicity of antineoplastic agents, without any deleterious effect on the anti-tumor activity of these agents.

In a first aspect, the invention provides new cardioprotectant rocaglaol derivatives of the formula (I) 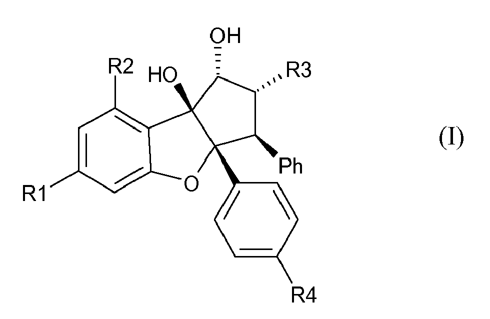 wherein
R³ is selected from the group consisting of -COOR⁵ and -CONH₂, wherein
R⁵ is (C₁-C₃)-alkyl, preferably methyl,
and wherein
R¹ is alkoxy, optionally substituted, preferably selected from the group consisting of methoxy and a group -O-(CH₂)ₙ-R¹⁰ wherein n is 1, 2, 3 or 4 and R¹⁰ is selected from the group consisting of hydroxyl, -NMe₂, -OCONMe₂, -OCONH₂ and morpholine;
R² is selected from the group consisting of hydrogen and (C₁-C₃)-alkoxy, preferably from hydrogen and methoxy; and,
R⁴ is halogen, preferably bromine, chloride, iodide, more preferably bromine,
or,
R¹ is alkoxy, optionally substituted, preferably selected from the group consisting of methoxy and a group -O-(CH₂)ₙ-R¹⁰ wherein n is 1, 2, 3 or 4 and R¹⁰ is selected from the group consisting of hydroxyl, -NMe₂, -OCONMe₂, -OCONH₂ and morpholine;
R² is hydrogen; and,
R⁴ is selected from the group consisting of hydrogen and alkoxy,
or,
R¹ is substituted alkoxy, preferably a group -O-(CH₂)ₙ-R¹⁰ wherein n is 1, 2, 3 or 4 and R¹⁰ is selected from the group consisting of hydroxyl, -NMe₂, -OCONMe₂, - OCONH₂ and morpholine;
R² is (C₁-C₃)-alkoxy, preferably methoxy; and
R⁴ is selected from the group consisting of hydrogen and alkoxy,
or any pharmaceutically acceptable salt thereof.

The present invention also provides a rocaglaol derivative of the formula (I), wherein
R³ is selected from the group consisting of hydrogen, -COOR⁵ and -CONR⁶R⁷, wherein
R⁵ is (C₁-C₃)-alkyl, preferably methyl, and
R⁶ and R⁷ are independently selected from the group consisting of hydrogen and (C₁-C₃)-alkyl, preferably from hydrogen and methyl; and wherein
R¹ is methoxy
R² is selected from the group consisting of hydrogen and (C₁-C₃)-alkoxy, preferably from hydrogen and methoxy; and,
R⁴ is bromine,
or any pharmaceutically acceptable salt thereof.

In a second aspect, the present invention concerns a rocaglaol derivative according to the invention as a medicament.

In another aspect, the present invention concerns a pharmaceutical composition comprising a cardioprotectant rocaglaol derivative according to the present invention and a pharmaceutically acceptable carrier and/or excipient.

The present invention also concerns a pharmaceutical composition comprising a rocaglaol derivative according to the present invention and an antineoplastic agent, preferably an anthracycline.

The present invention further concerns a product containing a rocaglaol derivative according to the present invention and an antineoplastic agent, preferably an anthracycline, as a combined preparation for simultaneous, separate or sequential use in the treatment of cancer.

In a further aspect, the present invention concerns a rocaglaol derivative of the formula (II) 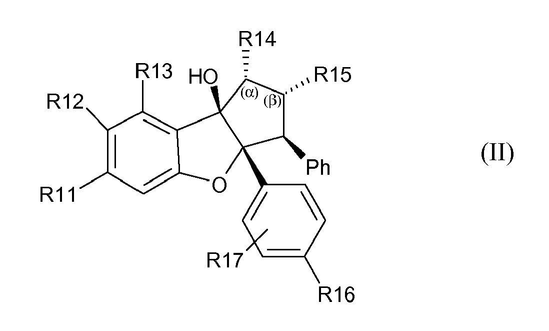 wherein
R¹¹ is alkoxy, optionally substituted; and R¹² is hydrogen; or,
R¹¹ and R¹² together form a -O-CH₂-O-unit;
R¹³ is selected from the group consisting of hydrogen and (C₁-C₃)-alkoxy, preferably from hydrogen and methoxy;
R¹⁴ is selected from the group consisting of hydroxyl, oxo group and -OCOR¹⁸, R¹⁸ being selected from the group consisting of (C₁-C₃)-alkyl and hydrogen, preferably from hydrogen and methyl;
R¹⁵ is selected from the group consisting of hydrogen, -COOR¹⁹, -CONR²⁰R²¹ and - CONH(CH₂)ₙOH, wherein n is 2, 3 or 4, wherein
R¹⁹ and R²¹ are independently selected from the group consisting of hydrogen and (C₁-C₃)-alkyl, preferably from hydrogen and methyl, and R²⁰ is selected from the group consisting of (C₁-C₃)-alkoxy, (C₁-C₃)-alkyl and hydrogen, preferably from hydrogen, methyl and methoxy, or
R²⁰ and R²¹ together form a 5 atom heterocycle, optionally substituted by a group -NHCO-(C₁-C₄)-alkyl;
or R¹⁴, R¹⁵and carbons (α) and (β) bearing R¹⁴ and R¹⁵ together form 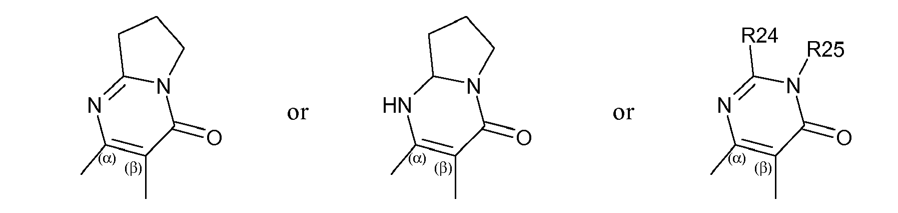 wherein R²⁴ and R²⁵ are independently selected from the group consisting of (C₁-C₃)-alkyl and hydrogen, preferably from hydrogen and methyl;
R¹⁶ is selected from the group consisting of hydrogen, (C₁-C₃)-alkoxy and halogen;
R¹⁷ is in ortho-position to R₁₆ and is selected from the group consisting of hydrogen, (C₁-C₃)-alkoxy and hydroxyl or form together with R¹⁷ a -O-CH₂-O-unit;
or any pharmaceutically acceptable salt thereof, for preventing or limiting the cardiotoxicity of an antineoplastic agent, preferably an anthracycline, more preferably doxorubicin.
In a preferred embodiment, the rocaglaol derivative is a rocaglaol derivative of formula (I) according to the present invention.

In another aspect, the present invention concerns a rocaglaol derivative of the formula (II) as described above or any pharmaceutically acceptable salt thereof as cardioprotectant agent. In a preferred embodiment, the rocaglaol derivative is a rocaglaol derivative of formula (I) according to the present invention.

In another aspect, the invention also concerns a method for preventing or limiting the cardiotoxicity of an antineoplastic agent in a subject comprising administering a therapeutically effective amount of a rocaglaol derivative of the formula (II) as described above or any pharmaceutically acceptable salt thereof to said subject. In a preferred embodiment, the rocaglaol derivative is a rocaglaol derivative of formula (I) according to the present invention.

### Legends of the Figures

Figure 1 shows a schematic representation of the synthesis of racemic bromo-demethoxy-rocaglaol (FL3).
Figure 2 shows a schematic representation of the synthesis of racemic rocaglaol (FL1) and its analogues (FL2 to FL4).
Figure 3 shows a schematic representation of the synthesis of racemic flavaglines FL5 to FL11.
Figure 4 shows the chemical structures of the rocaglaol (FL1) and the rocaglaol derivatives FL2-11.
Figure 5 is a graph that shows the inhibition of cell proliferation of HepG2 cells exposed to FL1 and FL3 with or without 150 nM doxorubicin. Experiments were performed in duplicate. HepG2 cells were treated with FL1 ( ) or FL3 (◊) alone or with doxorubicin (FL1 : ■, FL3:◆) for 72 h hours, and cell proliferation was measured by MTS assay. Experiments were performed in duplicate (n=3).
Figures 6A and 6B show the cardioprotective effect of FL1 and FL3 on apoptosis induced by doxorubicin in H9c2 cells. A. These two graphs represent the relative total apoptotic cells obtained with doxorubicin alone or in combination with FL1 (Figure 6A) or FL3 (Figure 6B), in comparison with the results obtained with the vehicle.
Figure 7 shows the cardioprotective effect of FL5, FL6, FL7 and FL8 on apoptosis induced by doxorubicin in H9c2 cells at different concentrations.
Figure 8 is a graph representing the inhibition of doxorubicin-induced caspase-3 activation by FL3 (1 nM) in H9c2 cells.
Figure 9 shows western-blot analysis of caspase-3 expression in cells exposed to doxorubicin or FL3 alone, or exposed to the combination doxorubicin and FL3.

### Detailed description of the invention

The inventors have surprisingly found that flavaglines, in particular rocaglaol and some of its derivatives, may act as cardioprotectant agents. These compounds are found to be able to prevent or limit the apoptosis induced by the cardiotoxicity of antineoplastic agents such as anthracyclines, while potentiating the anti-tumor efficacy of these molecules.

Flavaglines are a family of natural compounds extracted from Asian plants of the genus *Aglaia* (Kim et al., 2006 and Proksch et al., 2001), comprising, for instance, rocaglaol, rocaglamide or silvestrol. Some of these molecules are known to inhibit the proliferation of tumor cells in a low nanomolar range, either by cytostatic (Hausott et al., 2004) or cytotoxic effects (Kim et al., 2007 and Mi et al., 2006), without displaying any significant toxicity on normal cells. Up to now, flavaglines have only been known to exhibit anticancer, immunosuppressive, neuroprotective (Fahrig et al., 2005), antifungal insecticide and antiinflammatory (EP 1 693 059) properties.

The present invention provides new cardioprotectant rocaglaol derivatives of the formula (I) 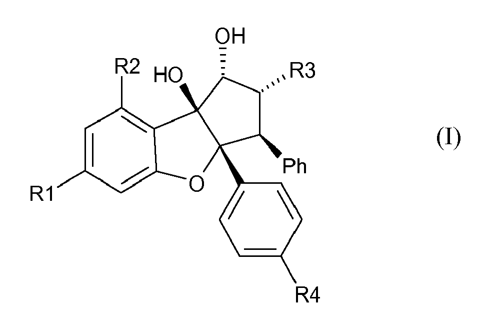 wherein
R¹ is alkoxy, optionally substituted, preferably selected from the group consisting of methoxy and a group -O-(CH₂)ₙ-R¹⁰ wherein n is 1, 2, 3 or 4 and R¹⁰ is selected from the group consisting of hydroxyl, -NMe₂, -OCONMe₂, -OCONH₂ and morpholin;
R² is selected from the group consisting of hydrogen and (C₁-C₃)-alkoxy, preferably from hydrogen and methoxy;
R³ is selected from the group consisting of -COOR⁵ and -CONH₂, wherein
R⁵ is (C₁-C₃)-alkyl, preferably methyl; and
R⁴ is halogen, preferably bromine, chloride, iodide, more preferably bromine,
or
wherein,
R¹ is alkoxy, optionally substituted, preferably selected from the group consisting of methoxy and a group -O-(CH₂)ₙ-R¹⁰ wherein n is 1, 2, 3 or 4 and R¹⁰ is selected from the group consisting of hydroxyl, -NMe₂, -OCONMe₂, -OCONH₂ and morpholin;
R² is hydrogen;
R³ is selected from the group consisting of -COOR⁵ and -CONH₂, wherein
R⁵ is (C₁-C₃)-alkyl, preferably methyl; and
R⁴ is selected from the group consisting of hydrogen, halogen and alkoxy,
or
wherein,
R¹ is substituted alkoxy, preferably a group -O-(CH₂)ₙ-R¹⁰ wherein n is 1, 2, 3 or 4 and R¹⁰ is selected from the group consisting of hydroxyl, -NMe₂, -OCONMe₂, -OCONH₂ and morpholine.
R² is selected from the group consisting of hydrogen and (C₁-C₃)-alkoxy, preferably from hydrogen and methoxy;
R³ is selected from the group consisting of -COOR⁵ and -CONH₂, wherein
R⁵ is (C₁-C₃)-alkyl, preferably methyl, and
R⁴ is selected from the group consisting of hydrogen, halogen and alkoxy,
or any pharmaceutically acceptable salt thereof.

In one embodiment, R¹ is selected from the group consisting of methoxy or O-(CH₂)ₙ-OH
wherein n is 1, 2 or 3, preferably R¹ is a methoxy;
R² is hydrogen;
R³ is selected from the group consisting of -COOR⁵ and -CONH₂, wherein
R⁵ is methyl; and
R⁴ is halogen or methoxy, preferably bromine or methoxy.

In another embodiment, R¹ is a substituted alkoxy -O-(CH₂)ₙ-R¹⁰ wherein n is 1, 2, 3 or 4 and R¹⁰ is selected from the group consisting of hydroxyl, -NMe₂, -OCONMe₂, -OCONH₂ and morpholine, preferably R¹⁰ is hydroxyl;
R² is hydrogen or methoxy, preferably hydrogen;
R³ is selected from the group consisting of -COOR⁵ and -CONH₂, wherein
R⁵ is (C₁-C₃)-alkyl, preferably methyl; and
R⁴ is halogen or methoxy, preferably bromine or methoxy.

In a particular embodiment, R¹ is a substituted alkoxy -O-(CH₂)ₙ-R¹⁰ wherein n is 2 and R¹⁰ is hydroxyl; R² is hydrogen; R³ is -COOR⁵, wherein R⁵ is methyl; and R⁴ is methoxy.

In another particular embodiment, R¹ is a substituted alkoxy -O-(CH₂)ₙ-R¹⁰ wherein n is 3 and R¹⁰ is hydroxyl; R² is hydrogen; R³ is -COOR⁵, wherein R⁵ is methyl; and R⁴ is methoxy.

In a more preferred embodiment, the present invention concerns a compound of formula (I)
wherein
R¹ is methoxy;
R² is selected from the group consisting of methoxy or hydrogen;
R³ is selected from the group consisting of -COOR⁵, and -CONH₂, wherein R⁵ is methyl; and
R⁴ is halogen, preferably bromine.
In a particular embodiment, R¹ is methoxy, R² is hydrogen, R³ is -CONH₂ and R⁴ is bromine.

In another preferred embodiment,
R¹ is methoxy;
R² is hydrogen;
R³ is -COOR⁵, wherein R⁵ is methyl; and
R⁴ is halogen or methoxy, preferably bromine or methoxy.

In a particular embodiment, R¹ is methoxy; R² is hydrogen; R³ is -COOR⁵, wherein R⁵ is methyl; and R⁴ is bromine.

In another particular embodiment, R¹ is methoxy; R² is hydrogen; R³ is -COOR⁵, wherein R⁵ is methyl; and R⁴ is methoxy.

In another aspect, the present invention also concerns a rocagloal derivative of the formula (I), wherein
R¹ is methoxy;
R² is selected from the group consisting of hydrogen and (C₁-C₃)-alkoxy, preferably from hydrogen and methoxy;
R³ is selected from the group consisting of hydrogen, -COOR⁵ and -CONR⁶R⁷, wherein
R⁵ is (C₁-C₃)-alkyl, preferably methyl, and
R⁶ and R⁷ are independently selected from the group consisting of hydrogen and (C₁-C₃)-alkyl, preferably from hydrogen and methyl; and
R⁴ is bromine,
or any pharmaceutically acceptable salt thereof.

In a preferred embodiment, R¹ and R² are methoxy, R³ is hydrogen and R⁴ is bromine.

In another embodiment, R¹ is methoxy; R² is hydrogen; R³ is selected from the group consisting of hydrogen and -CON(CH₃)₂; and R⁴ is bromine.

In a preferred embodiment, R¹ is methoxy; R² is hydrogen; R³ is -CON(CH₃)₂ and R⁴ is bromine.

In another preferred embodiment, R¹ is methoxy; R² is hydrogen; R³ is hydrogen and R⁴ is bromine.

As used herein, a "cardioprotectant agent" is an agent that protects cardiomyocytes from damages, in particular from apoptosis.
The term "alkoxy", as used herein, corresponds to an alkyl group bonded to the molecule by an -O- (ether) bond. (C₁-C₃)-alkoxy groups include methoxy, ethoxy, propyloxy, and isopropyloxy.
The term "alkyl" as used herein, refers to a univalent radical containing only carbon and hydrogen atoms arranged in a chain. (C₁-C₃)-alkyl groups include methyl, ethyl, propyl, or isopropyl. The term "Me" as used herein, refers to a methyl group.
The term "morpholin" as used herein, refers to a heterocycle of formula -O(CH₂CH₂)₂NH with both amine and ether functional groups.
The term "heterocycle" as used herein, refers to a group containing a ring structure comprising one or more heteroatoms, such as sulfur, oxygen or nitrogen. 5 atom heterocycles include, for example, furan, pyrrole and oxazole.
The term "pharmaceutically acceptable salt" refers to salts which are non-toxic for a patient and suitable for maintaining the stability of a therapeutic agent and allowing the delivery of said agent to target cells or tissue. Pharmaceutically acceptable salts are well known in the art (Berge et al., 1977). These salts can be prepared in situ during the final isolation and purification of the rocaglaol derivatives, or separately by reacting the free base function with a suitable organic acid. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, 3-phenylpropionate, phosphate, picrate, pivalate, propionate, stearate, succinate, sulfate, tartrate, thiocyanate, p-toluenesulfonate, undecanoate, valerate salts, and the like.

The present invention also concerns a rocaglaol derivative of the formula (I) according to the invention, as described above, as a medicament, in particular as cardioprotectant agent, and more particularly as cardioprotectant agent against cardiotoxicity of an antineoplastic agent.

These rocaglaol derivatives can be synthesized by any process known by the man skilled in the art. Such processes are described, for example, in the patent applications DE 19934952 and WO 2005/092876 or in the articles of Diedrichs et al. (Diedrichs et al., 2005), Gerard et al. (Gerard et al., 2004) and Dobler et al. (Dobler et al., 2001). As illustrative example, the synthesis of two of these rocaglaol derivatives, namely bromo-demethoxy-rocaglaol (FL3) and methyl bromo-didemethoxy-rocaglate (FL5), is detailed in the present application in examples 1 and 2.

The present invention also concerns a pharmaceutical composition comprising a cardioprotectant rocaglaol derivative of formula (I) according to the present invention and a pharmaceutically acceptable carrier and/or excipient.

The pharmaceutical composition comprising the rocaglaol derivative of formula (I) according to the present invention is formulated in accordance with standard pharmaceutical practice (see, e.g., Remington: The Science and Practice of Pharmacy (20th ed.), ed. A. R. Gennaro, Lippincott Williams & Wilkins, 2000 and Encyclopedia of Pharmaceutical Technology, eds. J. Swarbrick and J. C. Boylan, 1988-1999, Marcel Dekker, New York) known by a person skilled in the art.

Possible pharmaceutical compositions include those suitable for oral, rectal, topical (including transdermal, buccal and sublingual), or parenteral (including subcutaneous, intramuscular, intravenous and intradermal) administration. For these formulations, conventional excipient can be used according to techniques well known by those skilled in the art.

The compositions for parenteral administration are generally physiologically compatible sterile solutions or suspensions which can optionally be prepared immediately before use from solid or lyophilized form. Adjuvants such as a local anesthetic, preservative and buffering agents can be dissolved in the vehicle and a surfactant or wetting agent can be included in the composition to facilitate uniform distribution of the active ingredient.

For oral administration, the composition can be formulated into conventional oral dosage forms such as tablets, capsules, powders, granules and liquid preparations such as syrups, elixirs, and concentrated drops. Non toxic solid carriers or diluents may be used which include, for example, pharmaceutical grades of mannitol, lactose, starch, magnesium stearate, sodium saccharine, talcum, cellulose, glucose, sucrose, magnesium, carbonate, and the like. For compressed tablets, binders, which are agents which impart cohesive qualities to powdered materials are also necessary. For example, starch, gelatine, sugars such as lactose or dextrose, and natural or synthetic gums can be used as binders. Disintegrants are also necessary in the tablets to facilitate break-up of the tablet. Disintegrants include starches, clays, celluloses, algins, gums and crosslinked polymers. Moreover, lubricants and glidants are also included in the tablets to prevent adhesion to the tablet material to surfaces in the manufacturing process and to improve the flow characteristics of the powder material during manufacture. Colloidal silicon dioxide is most commonly used as a glidant and compounds such as talc or stearic acids are most commonly used as lubricants.

For transdermal administration, the composition can be formulated into ointment, cream or gel form and appropriate penetrants or detergents could be used to facilitate permeation, such as dimethyl sulfoxide, dimethyl acetamide and dimethylformamide.

For transmucosal administration, nasal sprays, rectal or vaginal suppositories can be used. The active compound can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate.

In a preferred embodiment, the pharmaceutical composition of the invention is suitable for parenteral administration.

Pharmaceutical composition according to the invention may be formulated to release the active drug substantially immediately upon administration or at any predetermined time or time period after administration.

Pharmaceutical composition according to the invention can comprise one or more cardioprotectant rocaglaol derivatives associated with pharmaceutically acceptable excipients and/or carriers. These excipients and/or carriers are chosen according to the form of administration as described above. Other active compounds can also be associated with rocaglaol derivatives of the present invention such as other cardioprotectant molecules or antineoplastic agents, such as anthracycline, preferably doxorubicin.

The present invention also concerns a product containing a rocaglaol derivative of formula (I) according to the invention and an antineoplastic agent, preferably an anthracycline, as a combined preparation for simultaneous, separate or sequential use in the treatment of cancer.

The present invention also concerns the use of a rocaglaol derivative of the formula (II) 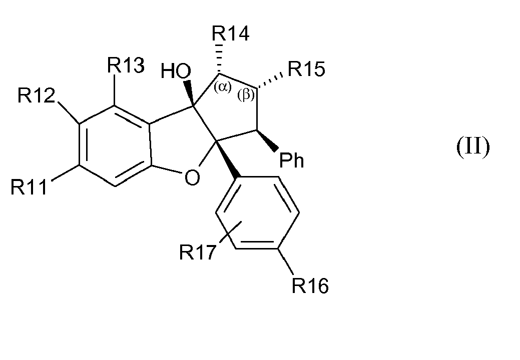 wherein
R¹¹ is alkoxy, optionally substituted; and R¹² is hydrogen;
or R¹¹ and R¹² together form a -O-CH₂-O-unit;
R¹³ is selected from the group consisting of hydrogen and (C₁-C₃)-alkoxy, preferably from hydrogen and methoxy;
R¹⁴ is selected from the group consisting of hydroxyl, oxo group and -OCOR¹⁸, R¹⁸ being selected from (C₁-C₃)-alkyl and hydrogen, preferably from hydrogen and methyl;
R¹⁵ is selected from the group consisting of hydrogen, -COOR¹⁹, -CONR²⁰R²¹ and - CONH(CH₂)ₙOH, wherein n is 2, 3 or 4, wherein
R¹⁹ and R²¹ are independently selected from hydrogen and (C₁-C₃)-alkyl, preferably from hydrogen and methyl, and R²⁰ is selected from the group consisting of (C₁-C₃)-alkoxy, (C₁-C₃)-alkyl and hydrogen, preferably from hydrogen, methyl and methoxy, or
R²⁰ and R²¹ together form a 5 atom heterocycle, optionally substituted by a group NHCO-(C₁-C₄)-alkyl;
or R¹⁴, R¹⁵ and carbons (α) and (β) bearing R¹⁴ and R¹⁵ together form 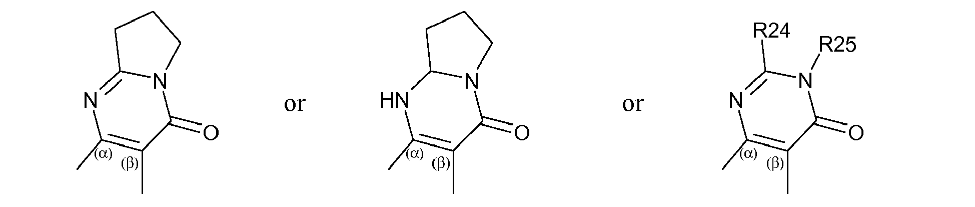 wherein R²⁴ and R²⁵ are independently selected from the group consisting of (C₁-C₃)-alkyl and hydrogen, preferably from hydrogen and methyl;
R¹⁶ is selected from the group consisting of hydrogen, (C₁-C₃)-alkoxy and halogen;
R¹⁷ is in ortho-position to R₁₆ and is selected from the group consisting of hydrogen, (C₁-C₃)-alkoxy and hydroxyl or form together with R¹⁷ a -O-CH₂-O-unit;
or any pharmaceutically acceptable salt thereof, for the manufacture of a medicament to prevent or limit the cardiotoxicity of an antineoplastic agent, preferably an anthracycline.

The present invention also concerns a rocaglaol derivative of the formula (II) as described above or any pharmaceutically acceptable salt thereof, for preventing or limiting the cardiotoxicity of an antineoplastic agent.

The present invention further concerns a method for preventing or limiting the cardiotoxicity of an antineoplastic agent in a subject comprising administering a therapeutically effective amount of a rocaglaol derivative of the formula (II) as described above or any pharmaceutically acceptable salt thereof to said subject.
The subject is any mammal, preferably a human being.

In a particular embodiment, the method of the invention for preventing or limiting the cardiotoxicity of an antineoplastic agent comprises administering 0.01 to 10 mg / kg of body weight / day of a rocaglaol derivative of the formula (II) as described above or any pharmaceutically acceptable salt thereof to said subject. Preferably, the method of the invention comprises administering 0.1 to 5 mg / kg of body weight / day of a rocaglaol derivative of the formula (II) as described above or any pharmaceutically acceptable salt thereof to said subject.

The term "cardiotoxicity", "cardiovascular toxicity" or "cardiotoxic side effect" means having a toxic effect on the heart, which includes cardiac events, such as but not limited to, mild blood pressure changes, thrombosis, electrocardiographic (ECG) changes, arrthymias, myocarditis, pericarditis, myocardial infarction (MI), cardiomyopathy, cardiac failure (left ventricular dysfunction or failure) and congestive heart failure (CHF). Considering the cardiotoxicity of antineoplastic agents, these side effects are essentially due to apoptosis of cardiomyocytes induced by such agents.

An "antineoplastic agent" is an agent with anti-cancer activity that inhibits or halts the growth of cancerous cells or immature pre-cancerous cells, kills cancerous cells or immature pre-cancerous cells, increases the susceptibility of cancerous or pre-cancerous cells to other antineoplastic agents, and/or inhibits metastasis of cancerous cells. These agents may include chemical agents as well as biological agents. Various antineoplastic agents can cause cardiovascular toxicity such as anthracyclines, cyclophosphamide, paclitaxel, fluorouracil, rituximab, arsenic trioxide, trastuzumab, thalidomide, etoposide, vinca alkaloids, pentastatin, cytarabine, interferons, busulfan and cisplatin.

Preferably, the antineoplastic agent belongs to the group of anthracyclines. In the present document, "anthracyclines" include but are not limited to, doxorubicin, daunorubicin, idarubicin, detorubicin, carminomycin, epirubicin, morpholinodoxorubicin, morpholinodaunorubicin, methoxymorpholinyldoxorubicin, substitutes, derivatives, and one or more combinations thereof. More preferably, the anthracycline molecule is doxorubicin.

As used herein, the term "to prevent or to limit the cardiotoxicity" means inhibiting or reducing cardiotoxic side effects of the antineoplastic agent administered to the subject, in particular, inhibiting or reducing the apoptosis of cardiomyocytes. The effect of the rocaglaol derivative on the apoptosis of cardiomyocytes can be assessed by any method known by the skilled person. For example, in the experimental section, the effects of compounds according to invention are assessed by measuring and comparing the apoptosis of H9c2 cardiomyocytes in absence and in presence of an antineoplastic agent and with a combination of an antineoplastic agent and a rocaglaol derivative of the invention. Results are expressed in percentage of apoptotic cells. Preferably, the apoptosis of cardiomyocyte in presence of a rocaglaol derivative of the invention is reduced by at least 5%, more preferably by at least 10% and the most preferably by at least 25%.

The present invention also concerns the use of a rocaglaol derivative of the formula (II) as described above, or any of its pharmaceutically acceptable salts, as cardioprotectant agent.

Preferably, the rocaglaol derivative is one of the formula (I) according to the invention, as described herein.

The amount of cardioprotectant rocaglaol derivative of the invention to be administered has to be determined by standard procedure well known by those of ordinary skill in the art. Physiological data of the patient (e.g. age, size, and weight) and the routes of administration have to be taken into account to determine the appropriate dosage. The cardioprotectant rocaglaol derivative of the invention may be administered as a single dose or in multiple doses.

In a preferred embodiment, the pharmaceutical composition comprises a therapeutically effective amount of the rocaglaol derivative of the invention.

By a "therapeutically effective amount" is intended an amount of cardioprotectant rocaglaol derivative of the invention administered to a patient that is sufficient to provide a cardioprotective effect, i.e. that is sufficient to protect cardiomyocytes from damages, in particular from apoptosis.

This therapeutically effective amount comprises a blood plasma concentration in a range of from 1 nM to 1 µM, preferably of from 15 nM to 100 nM or from 18 nM to 90 nM, more preferably of from 20 nM to 70 nM, most preferably of from 23 nM to 50 nM, and even most preferably of from 25 nM to 30 nM. Then, it is to be understood that the total amount of the rocaglaol derivative of the invention may vary depending on the volume of blood plasma of the patient. Suitable means and measures to determine the therapeutically effective amount are available to the person skilled in the art.

In a particular embodiment, the pharmaceutical composition according to the invention comprises 0.1 mg to 1 g of the rocaglaol derivative of the invention. In another particular embodiment, the pharmaceutical composition according to the invention comprises 0.7 to 700 mg of the rocaglaol derivative of the invention, preferably 0.7 to 350 mg of the rocaglaol derivative of the invention.

Cardioprotectant rocaglaol derivatives of the invention can be used in combination with other cardioprotective molecules or with antineoplastic agents. In this case, rocaglaol derivatives and the other active molecules can be administered simultaneously or consecutively.

Accordingly, the present invention discloses a pharmaceutical composition comprising a rocaglaol derivative of formula (II), preferably of formula (I), and an antineoplastic agent. It also concerns a product containing a rocaglaol derivative of formula (II), preferably of formula (I) and an antineoplastic agent as a combined preparation for simultaneous, separate or sequential use in the treatment of cancer. In a preferred embodiment, the antineoplastic agent is an anthracycline, in particular doxorubicin.

The following examples are given for purposes of illustration and not by way of limitation.

### Examples

### Example 1: Synthesis of bromo-demethoxy-rocaglaol (FL3)

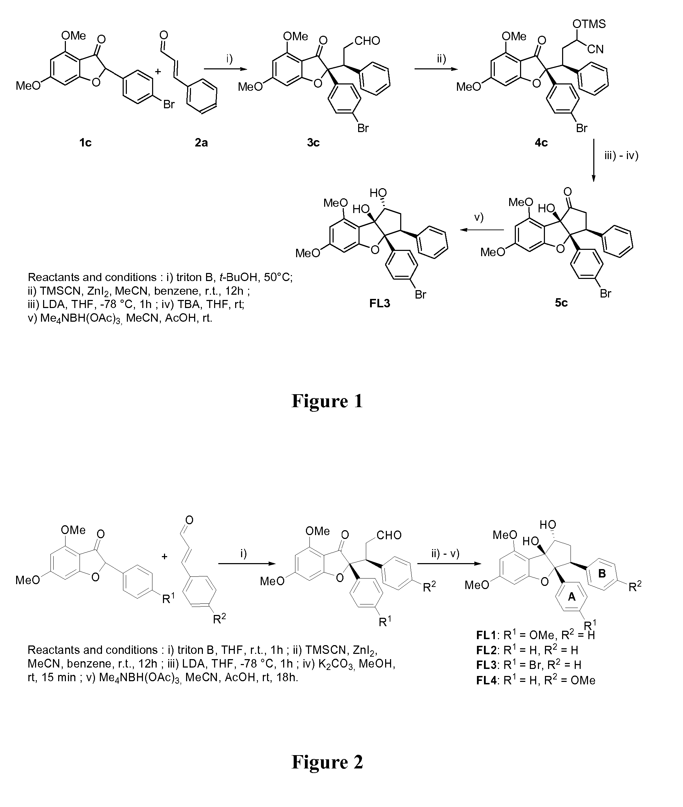

The synthesis of bromo-demethoxy-rocaglaol (FL3) is represented on Figure 1. The different steps are described below.

### (S)-3-((R)-2,3-dihydro-4,6-dimethoxy-2-(4-bromophenyl)-3-oxobenzofuran-2-yl)-3-phenylpropanal (3c)

A suspension of benzofuranone **1c** (4.7 g, 13.5 mmol) in *t*-BuOH (300 ml) was heated to 50°C under argon. Benzyltrimethylammonium hydroxide in MeOH (40%, 306 µL, 0.73 mmol) and, immediately after, cinnamaldehyde **2a** (3.40 ml, 27.0 mmol), were added. The mixture was stirred for 2 h at 50°C, cooled to room temperature (rt), concentred and acidified with HCl 1 M (30 ml), extracted with CH₂Cl₂, dried over MgSO₄ and concentrated to dryness. Purification of the resulting yellow solid residue by chromatography (Et₂O-Pentane 6:4) yielded 1.94 g (33%) of cis-ketoaldehyde **3c** as a white solid: *R*_{f} 0.4 (Et₂O/Hept 9:1). NMR ¹H (300 MHz, CDCl₃): 2.61 (1H, ddd, *J* = 0.9, 4.0, 17.3 Hz), 3.07 (1H, ddd, *J* = 2.30, 10.9, 17.3 Hz), 3.70 (3H, s), 3.85 (3H, s), 4.19 (1H, dd, *J* = 4.0, 10.9 Hz), 5.81 (1H, d, *J* = 1.8 Hz), 6.21 (1H, d, *J* = 1.8 Hz), 7.08-7.17 (3H, m), 7.29-7.32 (2H, m), 7.49, 7.63 (4H, AA'BB', *J* = 8.7 Hz), 9.41 (1H, d, *J* = 1.1 Hz). NMR ¹³C (75 MHz, CDCl₃): 44.0; 46.8; 55.9; 55.9; 88.4; 93.0; 103.7; 122.6; 126.9; 127.5; 128.2; 129.5; 131.8; 132.0; 135.6; 136.3; 159.1; 169.9; 174.1; 194.1; 200.0. IR (thin film): 2725, 1720, 1699, 1617, 1590, 1154, 747 cm⁻¹. HR-MS calcd for C₂₅H₂₁BrLiO₅ : 487.0732; found : 487.0727 (-0.02 ppm).

### (S)-3-((R)-2,3-dihydro-4,6-dimethoxy-2-(4-bromophenyl)-3-oxobenzofuran-2-yl)-3-phenyl-2-[(trimethylsilyl)oxy]-propanenitrile (4c)

Acetonitrile (12 mL) was added at room temperature under argon to aldehyde **3c** (1.2 g, 2.5 mmol). Trimethylsilyl cyanide (744 mg, 7.5 mmol) was added dropwise and immediately after zinc iodide (10 mg) was added at room temperature under argon. The resulting mixture was stirred for 1 hour, filtered and concentred. Cyanohydrin **4c** (1.74 g) was used in the next step without purification. *R_{f}* 0.5 (Et₂O/Hept 8:2). NMR ¹H (300 MHz, CDCl₃): 7.47-7.60 (4H, m); 7.13-1.23 (5H, m) ; 6.21 (1H, t, *J* = 2.3 Hz); 5.79 (1H, t, *J* = 2.3 Hz); 3.87-3.89 (1H, m); 3.83 (3H, s); 3.70-3.76 (1H, m); 3.66 (3H, s); 2.30-2.38 (2H, m); -0.01 (9H, d, J= 4.5 Hz).

### Tricyclic Ketone (5c)

LDA (2.7 mmol, 0.6 M) was added dropwise at -78°C under argon to a solution of protected cyanohydrin **4c** (1.43 g, 2.46 mmol) in dry THF (12 mL). After stirring for 2 hours at -78°C the solution was heated to -50°C for 10 min. The reaction was quenched by the addition of saturated aqueous ammonium chloride (15 mL). Standard extractive work-up (CH₂Cl₂) gave a yellow solid (1.49 g).This solid was directly treated with tetra-n-butylammonium fluoride (2.7 mL, 1M in THF) added dropwise at room temperature in dry THF (10 mL). The solution was stirred for 4 hours and she was quenched by addition of methanol. Standard extractive work-up (AcOEt) and purification by flash chromatography (Et₂O) afforded tricyclic ketone **5c** (240 mg, 20 %) as a white solid: *R_{f}* 0.35 (Et₂O/Hept 8:2). NMR ¹H (300 MHz, CDCl₃): 2.96-3.09 (2H, m), 3.21 (1H, br s), 3.81 (3H, s), 3.84 (3H, s), 3.90 (1H, dd, *J* = 10.2, 12.1 Hz), 6.1 (1H, d, *J* = 1.9 Hz), 6.33 (1H, d, *J* = 1.9 Hz), 6.89-6.94 (4H, m), 7.10-7.12 (3H, m), 7.24-7.27 (2H, m). NMR ¹³C (75 MHz, CDCl₃): 39.6; 48.6; 55.6; 55.7; 88.6; 89.7; 92.8; 100.8; 106.2; 121.7; 127.1; 127.8; 128.0; 128.3; 130.7; 133.0; 136.6; 158.4; 160.9; 164.8; 210.3. IR (thin film): 3477, 2942, 2841, 1750, 1621, 1597, 1149 cm⁻¹. HR-MS calcd for C₂₅H₂₁Br₁Na₁O₆ : 503.0470; found : 503.0465 (-0.99 ppm).

### Racemic bromo-demethoxy-rocaglaol (FL3)

Acetonitrile (1.8 mL) was added at room temperature under argon to tetramethylammonium triacetoxyborohydride (828 mg, 3.15 mmol) followed by the addition of glacial acetic acid (1.8 mL). After stirring for 30 min at room temperature a solution of ketone **5c** (170 mg, 0.354 mmol) in dry acetonitrile (4.5 mL) was added dropwise. The resulting mixture was stirred overnight at room temperature. The reaction was quenched by the addition of saturated aqueous ammonium chloride (40 mL). Standard extractive work-up (AcOEt) and purification by flash chromatography (Et₂O) afforded **FL3** (103 mg, 60 %) as a white solid: *R_{f}* .25 (Et₂O/ Hept 8:2). NMR ¹H (300 MHz, CDCl₃): 1.89 (1H, br s), 2.14 (1H, dd, *J* = 7.0, 13.3 Hz), 2.67 (1H, ddd, *J* = 6.4, 13.9, 13.9 Hz), 3.27 (1H, br s), 3.83 (3H, s), 3.86 (3H, s), 3.99 (1H, dd, *J* = 6.5, 14.0 Hz), 4.77 (1H, d, *J* = 6.0 Hz), 6.1 (1H, d, *J* = 1.8 Hz), 6.28 (1H, d, *J* = 1.8 Hz), 6.95-6.98 (2H, m), 7.05-7.12 (5H, m), 7.23-7.26 (2H, m). NMR ¹³C (75 MHz, CDCl₃): 36.1; 53.2; 55.6; 55.7; 79.0; 89.4; 92.5; 94.8; 103.1; 107.4; 121.4; 126.4; 127.7; 127.9; 129.4; 130.2; 134.1; 138.1; 156.8; 160.6; 163.9. IR (thin film): 3486, 2942, 2841, 1624, 1598, 1147 cm⁻¹. HR-MS calcd for C₂₅H₂₃Br₁K₁O₆ : 521.0366; found : 521.0360 (-2.85 ppm).

### Example 2: Synthesis of methyl bromo-didemethoxy-rocaglate (FL5)

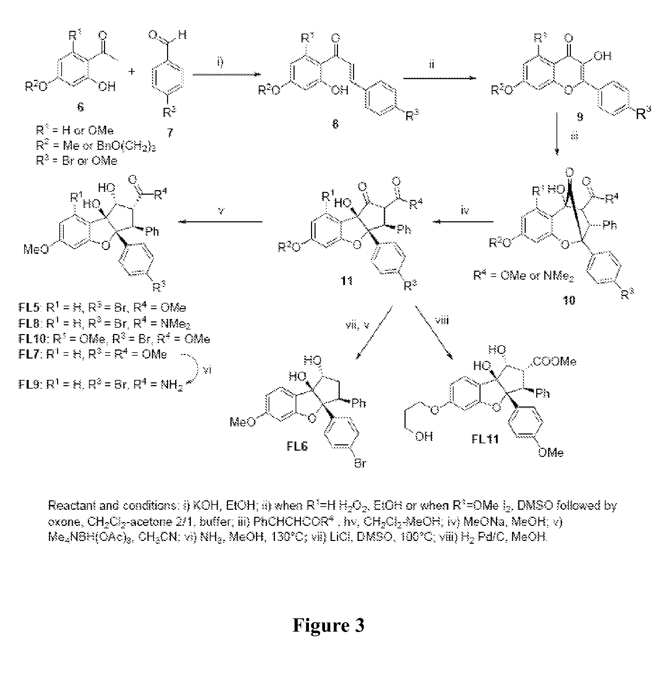

The synthesis of methyl bromo-didemethoxy-rocaglate (FL5) represented on Figure 3 is based on Porco's Strategy (Gerard et al., 2004). The different steps are described below.

### (E)-3-(4-bromophenyl)-1-(2-hydroxy-4-methoxyphenyl)prop-2-en-1-one (Figure 3 (8): R¹ = H, R² = Me, R³ = Br)

To a solution of 10.2 g (0.062 mol) of 2'-hydroxy-4'-methoxyacetophenone in 150 mL of methanol, were successively added 13.8 g (4 eq, 0.25 mol) of potassium hydroxide and 11.4 g (1 eq, 0.062 mol) of 4-bromobenzaldehyde. The solution was heated overnight at 60°C, cooled to 0°C in an ice bath and acidified to pH 2 with concentrated HCl. The precipitate was filtered, washed with 100 mL of water and dried *in vacuo* to afford 20.4 g (99%) of chalcone 8. NMR ¹H (300 MHz, CDCl₃): 3.83 (3H, s,), 6.45 (2H, m), 7.50 (5H, m), 7.77 (2H, m), 13.33 (1H, s). NMR ¹³C (75 MHz, CDCl₃): 55.8, 101.3, 108.1, 114.2 , 121.1, 125.1, 130.0, 131.4, 132.4, 133.9, 143.1, 166.6, 167.0, 191.7.

### 2-(4-bromophenyl)-3-hydroxy-7-methoxy-4H-chromen-4-one (Figure 3 (9): R¹ = H, R² = Me, R³ = Br)

To a solution of 10.0 g (1 eq, 0.030 mol) of chalcone 8 in 100 mL of methanol, were added 10.1 g (6 eq, 0.18 mol) of potassium hydroxide. The solution was heated at 60°C, and when it became red, 12.3 mL (4 eq, 0.12 mol) of hydrogene peroxide were slowly added. The solution was stirred for 15 min at r.t and cooled to 0°C in an ice bath. The mixture was then acidified to pH 2 with concentrated HCl. The precipitate was filtered, washed with 100 mL of water and dried *in vacuo* to afford 7.30 g (70%) of 3-hydroxyflavone 9 as a yellow solid. NMR ¹H (300 MHz, CDCl₃): 3.92 (3H, s₂), 6.92 (1H, d, J = 2.2 Hz), 6.98 (1H, dd, J = 2.2; 8.8 Hz), 7.63 (2H, d, J = 8.8 Hz), 8.11 (3H, m). NMR ¹³C (75 MHz, CDCl₃): 56.0, 100.2, 114.7, 115.1, 122.9, 126.0, 129.1, 130.6, 131.4, 139.1, 143.2, 156.4, 163.7, 172.3.

### Cyclopenta[bc]benzopyran (Figure 3 (10): R¹ = H, R² = Me, R³ = Br)

A solution of 3-hydroxyflavone 9 (1 g, 1 eq, 3 mmol) and methyl cinnamate (5.15 g, 10 eq, 30 mmol) in 90 mL of a mixture of dichloromethane/methanol (3/1) in a pyrex tube was degassed with argon for 5 min, and irradiated for 15h at 0°C using an Iwasaki 400 W mercury lamp. The solution was concentrated in vacuo and purified by flash chromatography (40/60 heptane/AcOEt) to afford 560 mg of cyclopenta[bc]benzopyrans as a white solid, which was heated at 65 °C for 4h in 20 mL of AcOEt. The solution was concentrated in vacuo to afford 560 mg (38%) of cyclopenta[bc]benzopyran 10 as a white solid. NMR ¹H (300 MHz, CDCl₃): 3.80 (3H, s), 3.82 (3H, m), 3.70 (1H, d, J = 8.3 Hz), 4.65 (1H, d, J = 8.3 Hz), 6.56 (2H, m), 7.19-7.40 (9H, m), 7.52 (1H, d, *J* = 8.4 Hz). NMR ¹³C (75 MHz, CDCl₃): 52.8, 54.4, 55.8, 60.9, 88.6, 97.8, 97.3, 101.9, 108.4, 117.0, 122.5, 125.7, 127.4, 128.5, 130.0, 130.8, 134.7, 139.3, 152.6, 161.7, 171.5, 191.5.

### Cyclopenta[b]tetrahydrobenzofuran (Figure 3 (11):, R¹ = H, R² = Me, R³ = Br)

To a solution of cyclopenta[bc]benzopyran 10 (400 mg, 1 eq, 0.8 mmol) in MeOH (75 mL) were added 78 mg (2,5 eq, 2.0 mmol) of NaH in MeOH (5 mL) at 0°C. The resulting solution was stirred for 20 min at 60°C. After quenching the reaction with saturated aqueous NH₄Cl, 40 mL of AcOEt was then added, and the organic layer was washed with water (2x20 mL) and brine (20 mL). The organic layer was dried over MgSO₄ and concentrated *in vacuo* to afford 370 mg of crude ketol (93%) as a white solide which was used without further purification. NMR ¹H (300 MHz, CDCl₃): 3.67 (3H, s), 3.87 (3H, s), 4.11 (1H, s), 4.54 (1H, s, H₆), 6.64 (1H, dd, *J* = 2.3, 8.4 Hz), 6.72 (1H, d, *J* = 2.3 Hz), 6.90-7.40 (10H, m).

### Methyl bromo-didemethoxy-rocaglate (FL5)

To a solution of 620 mg (6 eq, 2.35 mmol) of Me₄NBH(OAc)₃ and 0.23 mL (10 eq, 3.93 mmol) of acetic acid in 20 mL of CH₃CN was added a solution of 200 mg (1 eq, 0.39 mmol) of the crude ketol product in 10 mL of CH₃CN. The resulting solution was stirred for 3 h at rt before being quenched with 20 mL of saturated aqueous NH₄Cl. The solution was then treated with 10 mL of a 3 M aqueous solution of sodium/potassium tartrate and stirred for 30 min. The aqueous solution was extracted with CH₂Cl₂ (2x30 mL). The combined organic layers were washed with brine, dried over MgSO₄ and concentrated *in vacuo.* HPLC purification afforded 100 mg (50%) of the corresponding endo product as a white solid. NMR ¹H (300 MHz, CDCl₃): 3.67 (3H, s), 3.87 (3H, s), 3.92 (1H, dd, *J* = 14, 6.2 Hz), 4.45 (1H, d, *J* = 14 Hz), 4.83 (1H, d, *J* = 6.2 Hz), 6.60 (2H, m), 6.90-7.15 (7H, m), 7.23 (2H, d, *J* = 8.6 Hz), 7.33 (1H, d, *J* = 8.1 Hz). NMR ¹³C (75 MHz, CDCl₃): 51.1, 52.8, 56.2, 79.5, 93.6, 97.3, 101.8, 109.1, 119.1, 122.2, 127.5, 128.3, 128.7, 129.8, 131.0, 134.4, 137.0, 160.9, 163.6, 172.0.

### Example 3: NMR characterization of rocaglaol derivatives FL6 to FL11

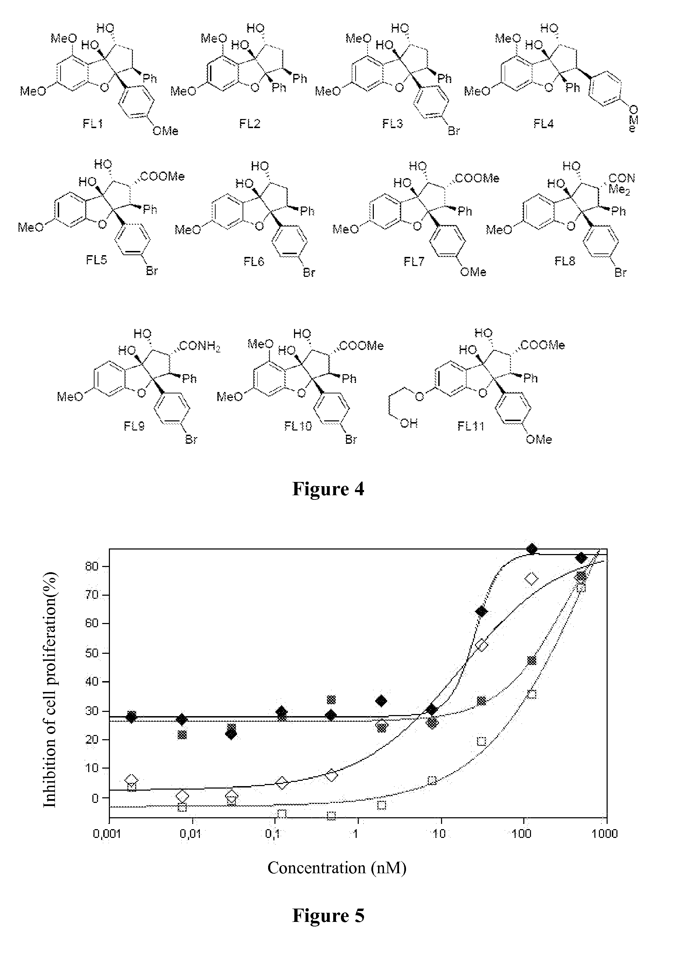

The structures of rocaglaol derivatives FL6 to FL11 are presented in Figure 4.

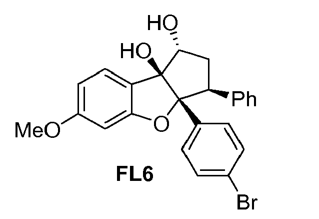

NMR ¹H (300 MHz, CDCl₃): 2.36 (1H, ddd, J = 2.8, 7.0, 13.5 Hz), 2.79 (1H, dt, J = 5.3, 13.4 Hz), 3.88 (3H, s), 4.27 (1H, dd, J = 6.9, 13.4 Hz), 4.71 (1H, dd, J = 2.7, 5.1 Hz), 6.64 (2H, m), 6.90-7.30 (10H, m). NMR ¹³C (75 MHz, CDCl₃): 36.9, 53.6, 55.7, 78.4, 94.1, 96.7, 102.8, 108.4, 118.6, 121.6, 126.3, 126.5, 128.0, 128.2, 129.3, 130.5, 134.8, 138.7, 160.8, 163.0.

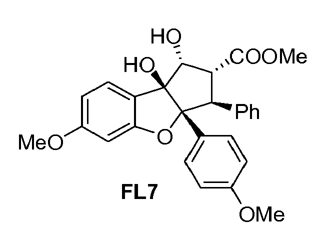

NMR ¹H (300 MHz, CDCl₃): 2.42 (1H, d, J = 5.4 Hz), 3.69 (3H, s), 3.70 (3H, s), 3.83 (3H, s), 3.93 (1H, dd, J = 5.4, 13.4 Hz), 4.43 (1H, d, J = 13.4 Hz), 4.83 (1H, t, J = 5.4 Hz), 6.60 (4H, m), 6.90-7.15 (7H, m), 7.31 (1H, d, J = 8.3 Hz). NMR ¹³C (75 MHz, CDCl₃): 51.1, 52.6, 55.4, 55.7, 56.0, 79.3, 93.0, 97.1, 101.9, 108.6, 113.2, 119.5, 126.9, 127, 127.5, 128.2, 128.4, 129.2, 137.4, 159.0, 160.9, 163.1, 172.0.

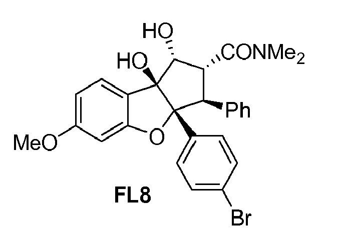

NMR ¹H (300 MHz, CDCl₃): 2.86 (3H, s), 3.19 (3H, s), 4.80 (3H, s), 4.06 (1H, dd, J = 7.0, 13.4 Hz), 4.40 (1H, d, J = 13.3 Hz), 4.77 (1H, d, J = 7.0 Hz), 6.55 (2H, m), 6.77 (2H, m), 7.02 (5H, m, H2), 7.24 (2H, m), 7.37 (1H, d, J = 7.9 Hz).

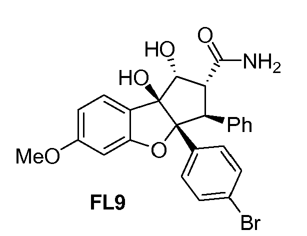

NMR ¹H (300 MHz, CDCl₃): 3.81 (3H, s, H19), 3.94 (1H, dd, J = 5.7, 13.5 Hz), 4.45 (1H, d, J = 13.5 Hz), 4.85 (1H, d, J = 5.6 Hz), 6.59 (2H, m), 6.90-7.30 (10H, m). NMR ¹³C (75 MHz, CDCl₃): 53.6, 55.7, 78.9, 93.1, 97.3, 101.4, 108.7, 118.8, 121.8, 127.2, 128.0, 128.6, 129.4, 130.5, 134.0, 136.6, 160.3, 163.0, 174.8.

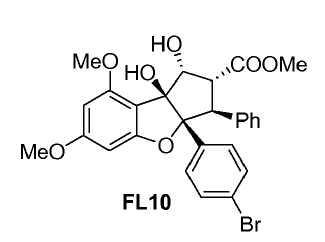

NMR ¹H (300 MHz, CDCl₃): 3.64 (3H, s), 3.82 (3H, s), 3.86 (3H, s), 3.92 (1H, dd, J = 14.1, 6.6 Hz), 4.33 (1H, d, J = 14.1 Hz), 5.01 (1H, d, J = 6.6 Hz), 6.12 (1H, d, J = 2.0 Hz), 6.27 (1H, d, J = 2.0 Hz), 6.87 (2H, m), 7.07 (5H, m), 7.25 (2H, d, J = 8.3 Hz). NMR ¹³C (75 MHz, CDCl₃): 50.4, 52.1, 55.1, 55.8, 60.5, 79.7, 89.6, 92.8, 93.8, 101.7, 107.6, 121.7, 126.9, 127.8, 128.0, 129.6, 130.3, 134.1, 136.7, 157.1, 160.7, 164.3, 170.5.

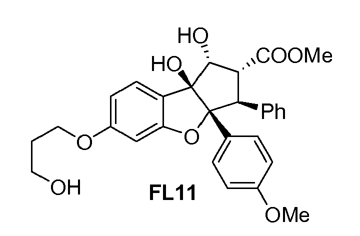

NMR ¹H (300 MHz, CDCl₃): 2.12 (2H, quint, *J* = 6.1 Hz) ; 3.60 (3H, s); 3.78 (3H, s); 4,21 (4H, m); 3,93 (2H, t, *J* = 5.7 Hz); 5.06 (1H, d, *J* = 7.3 Hz); 6.71 (6H, m); 7.11 (5H, m); 7.47 (1H, d, *J* = 8.9 Hz). NMR ¹³C (75 MHz, CDCl₃): 31.9, 49.5, 52.0, 55.1, 55.4, 60.4, 65.9, 78.9, 96.9, 97.5, 98.8, 108.8, 112.6, 127.2, 127.3, 127.9, 128.0, 128.1, 129.2, 136.3, 158.6, 161.6, 171.0.

### Example 4 : Effect of rocaglaol and its derivatives on human cancer cell viability and on doxorubicin cardiotoxicity

### Synthesis of racemic rocaglaol and its derivatives

Racemic rocaglaol (FL1) and its derivatives (FL2-4) were synthesised by the approach developed by Dobler and collaborators (Dobler et al., 2001) (Figures 2 and 3). The synthesis of FL3 and FL5 are detailed in examples 1 and 2. Rocaglaol derivatives FL5-11 were synthesized according to the strategy of Porco and collaborators (Gerard et al., 2004) (Figure 3).

### Cell lines

The human cell lines KB (epidermoid carcinoma) and HepG2 (hepatocarcinoma) were obtained from ECACC (Salisbury, UK) and grown in D-MEM medium supplemented with 10% fetal calf serum, in the presence of penicilline, streptomycine and fungizone in 75 cm² flask under 5% CO2.
The human cell lines HCT116 (colon adenocarcinoma) and HCT15 (colon adenocarcinoma) were obtained from ECACC (Wiltshire, UK).
The human cell lines HL60 (promyeocytic leukaemia) and MCF7 (breast adenocarcinoma) were obtained from ATCC and from Dr Kassack from the University of Bonn in Germany, respectively.

The cell lines HCT116, HCT 15, MCF7 and HL60 were grown in RPMI medium.
Resistant MCF7 (MCF7R) cells were obtained by prolonged treatment with doxorubicin.
H9c2 cells (rat heart myoblast) were obtained from ATCC and grown in D-MEM medium supplemented with 10% foetal calf serum.

### Method for measuring inhibition of cell proliferation: MTS assay

Cells were plated in 96-well tissue culture plates in 200 µl medium and cultured during 24h before to be treated with FL1, FL2, FL3 or FL4 at a range of 0.5 nM to 10 µM dissolved in DMSO using a Biomek 2000 (Beckman). Controls received the same volume of DMSO (1% final volume). After a 72h exposure, MTS reagent (Promega) was added and incubated for 3h at 37°C. The absorbance was then monitored at 490 nm and results were expressed as the value (OD490 treated/OD490 control)×100 corresponding to the percentage of inhibition of cell proliferation. For IC50 determinations (50% of inhibition of cell proliferation), experiments were performed in separate duplicate.

### Effect of rocaglaol (FL1) and its derivatives (FL2, FL,3 and FL4) on human cancer cell viability

The effect of rocaglaol and its derivatives was determined on human cancer cell lines by MTS assay after a 72 h treatment. As shown in table 1, FL1 (racemic rocaglaol) reduced the cell proliferation and viability more effectively than the anticancer drug doxorubicin on a panel of human cancer cell lines. Effects were observed in the low nanomolar range. Removing the methoxy group present on ring A decreased potency more than 1 000 times (compound FL2). Conversely, the replacement of the methoxy group on ring A by a bromine atom improved the cytotoxicity on all these cancer cell lines (compound FL3). The rank of activity (H <<MeO<Br) suggests a preference for an hydrophobic substituent in the para position of ring A. On contrary, introducing a methoxy on the para position of ring B was detrimental for the cytotoxicity (FL4).

**Table 1: Inhibition of cell proliferation by flavaglines FL1 to FL4 on various human cancer cell lines (IC₅₀, nM).**

| Cell lines | FL1 | FL2 | FL3 | FL4 | Doxorubicin |
|---|---|---|---|---|---|
| KB | 2 | 2500 | <1 | >10000 | 1 |
| MCF7 | 4 | 10000 | 1 | NI | 12 |
| MCF7R | 4 | 10000 | <1 | >10000 | 58 |
| HCT116 | 6 | 6000 | <1 | >10000 | 6 |
| HCT15 | 6 | 8000 | 1 | NI | 81 |
| HepG2 | 70 | >10000 | 4 | NI | 240 |
| HL60 | 3 | 6000 | <1 | >10000 | 13 |

### Rocaglaol (FL1) and rocaglaol derivatives (FL2, FL3 and FL4) have not any deletetious effect on the doxorubicin cytotoxicity

To test the cytotoxic effect of combination of FL1 or FL3 with doxorubicin, the inventors selected HepG2 cells of hepatocellular carcinoma which display a low sensitivity toward clinically used anticancer drugs.
The inhibition of HepG2 cell proliferation with FL1, FL3 or doxorubicin alone and with doxorubicin associated with FL1 or FL3 is shown Figure 5. These results demonstrated that rocaglaol or rocaglaol derivatives have not any deleterious effect on doxorubicin cytotoxicity, on contrary, they strengthen this activity on cancer cells.

### Effect of rocaglaol and its derivatives on doxorubicin cardiotoxicity

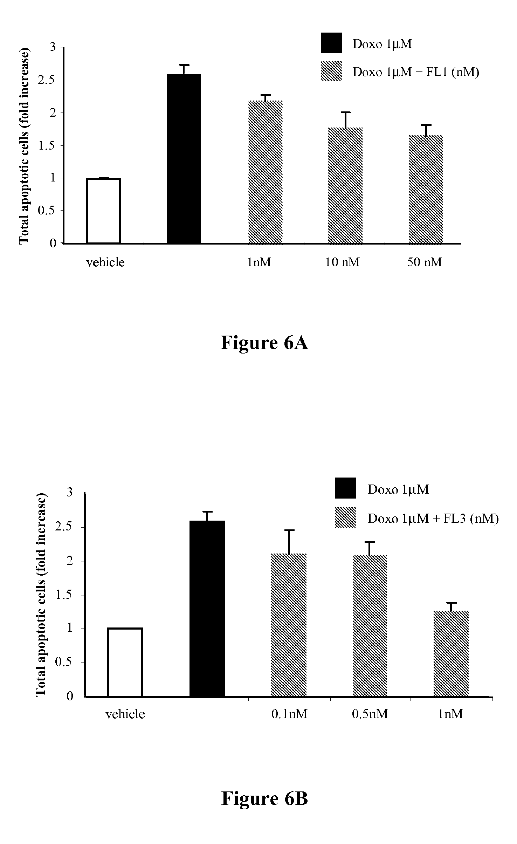

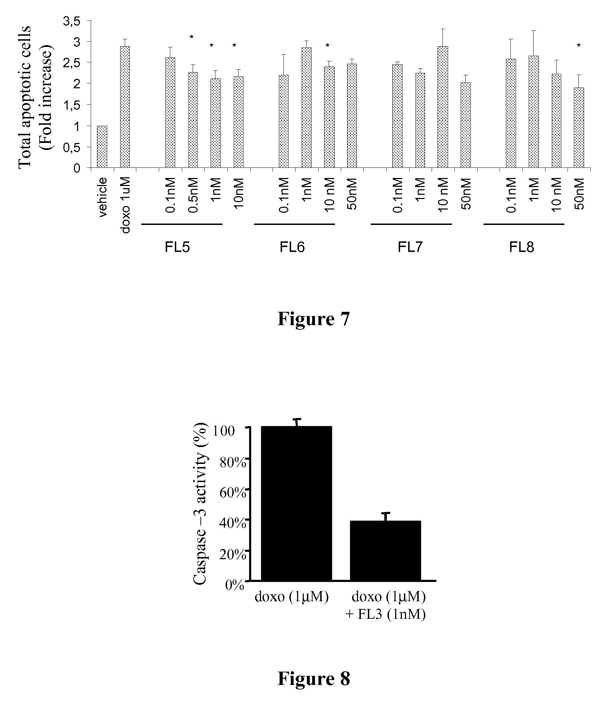

Apoptosis was induced in H9c2 cardiomyocytes by doxorubicin and detected by FACS analysis after labeling the cells with annexin and PI. Incubation of the cells with 1 µM doxorubicin for 14h induced 32% apoptosis (total apoptotic cells). It was observed that preincubation of H9c2 cells with different concentrations of rocaglaol and its derivatives significantly reduced the apoptosis induced by doxorubicin (Figures 6 and 7).
The most active compound, FL3, inhibited doxorubicin-induced apoptosis in a concentration-dependent manner reaching its maximum at 1 nM (Figure 6B). At this concentration, FL3 diminished by 70 % the apoptosis induced by 1 µM doxorubicin (Figure 8).

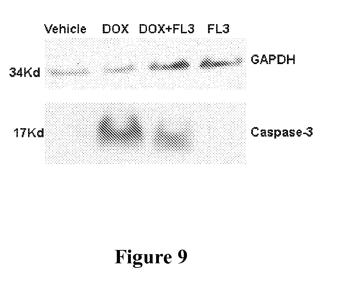

Western blot analysis with antibody specific for the active form of caspase 3 (Chemicon International, ref AB3623) revealed that FL3 reduced doxorubicin-mediated caspase-3 activity. Indeed, H9c2 cardiomyocytes incubated for 14h with 1 µM doxorubicin plus 1 nM FL3 failed to activate the cleavage of pro-caspase 3 in caspase 3 (Figure 9).

### Conclusion

These experiments demonstrated that rocaglaol and its derivatives according to the present invention are able to prevent or limit cardiomyocyte apoptosis, in particular doxorubicin-induced apoptosis.

### References

Berge, et al. J. Pharmaceutical Sciences, 1977, 66: 1-19.
Bueno O.F., et al. Circ Res. 2004; 94: 91-9.
Diedrichs N., et al. Eur J Org Chem. 2005, 9, 1731-1735.
Dobler M.R., et al. Tetrahedron Lett. 2001;42: 8281-8284.
Fahrig T., et al. Mol Pharm. 2005 ; 67 : 1544-1555.
Gerard B., et al. J Am Chem Soc 2004 ; 27 : 126: 13620-1.
Hausott B, et al. Int J Cancer. 2004 ; 109:933-940.
Ito T, et al. J Biol Chem. 2007; 282 : 1152-60.
Karmazyn M., et al. Can. J. Physiol. 1991, 69:719-730.
Kim S, et al. Anticancer Res 2007;27:2175-83.
Kim S, et al. Anticancer Agents Med Chem 2006;6:319-45.
Mi Q, et al. Anticancer Research 2006, 26:947-952.
Pai V.B., et al. Drug Saf. 2000;22:263-302.
Proksch P, et al. Curr Org Chem 2001;5:923-38.
Steinherz L.J., et al. Med. Pediatr. Oncol. 1995;24:352-361.
Swain S.M., et al. Cancer. 2003;97:2869-2879.
Van Dalen E.C., et al. Cochrane Database Syst Rev. 2005 Jan 25;(1):CD003917.
Wouters, K.A. et al. Br JHaematol. 2005 ; 131 :561-78.

## Claims

1. A rocaglaol derivative of the formula (II) 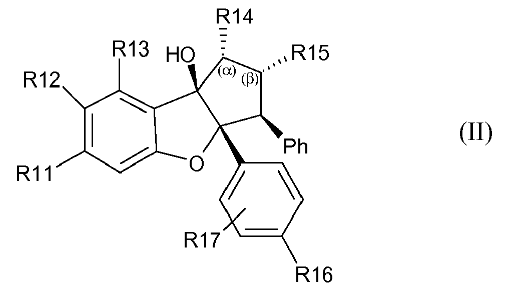 wherein
R¹¹ is alkoxy, optionally substituted;
R¹² is hydrogen;
or R¹¹ and R¹² together form a -O-CH₂-O-unit;
R¹³ is selected from hydrogen and (C₁-C₃)-alkoxy, preferably from hydrogen and methoxy;
R¹⁴ is selected from hydroxyl, oxo group and -OCOR¹⁸, R¹⁸ being selected from (C₁-C₃)-alkyl and hydrogen, preferably from hydrogen and methyl;
R¹⁵ is selected from hydrogen, -COOR¹⁹, -CONR²⁰R²¹ and -CONH(CH₂)ₙOH, wherein n is 2, 3 or 4, wherein
R¹⁹ and R²¹ are independently selected from hydrogen and (C₁-C₃)-alkyl, preferably from hydrogen and methyl, and R²⁰ is selected from the group consisting of (C₁-C₃)-alkoxy, (C₁-C₃)-alkyl and hydrogen, preferably from hydrogen, methyl and methoxy, or
R²⁰ and R²¹ together form a 5 atom heterocycle, optionally substituted by a group NHCO-(C₁-C₄)-alkyl;
or R¹⁴, R¹⁵ and carbons (α) and (β) bearing R¹⁴ and R¹⁵ together form 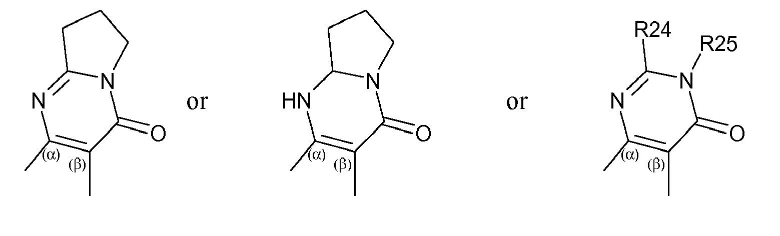 wherein R²⁴ and R²⁵ are independently selected from the group consisting of (C₁-C₃)-alkyl and hydrogen, preferably from hydrogen and methyl;
R¹⁶ is selected from hydrogen, (C₁-C₃)-alkoxy and halogen;
R¹⁷ is in ortho-position to R₁₆ and is selected from hydrogen, (C₁-C₃)-alkoxy and hydroxyl or form together with R¹⁷ a -O-CH₂-O-unit;
or any pharmaceutically acceptable salt thereof, for preventing or limiting the cardiotoxicity of an antineoplastic agent, preferably an anthracycline, more preferably doxorubicin.

2. Rocaglaol derivative of the formula (I) 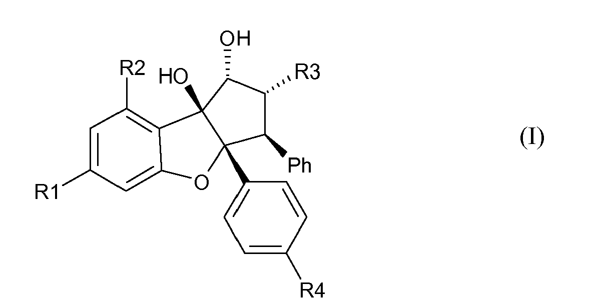 wherein
R³ is selected from the group consisting of -COOR⁵ and -CONH₂, wherein
R⁵ is (C₁-C₃)-alkyl, preferably methyl;
and wherein
R¹ is alkoxy, optionally substituted, preferably selected from the group consisting of methoxy and a group -O-(CH₂)ₙ-R¹⁰ wherein n is 1, 2, 3 or 4 and R¹⁰ is selected from the group consisting of hydroxyl, -NMe₂, -OCONMe₂, -OCONH₂ and morpholine;
R² is selected from the group consisting of hydrogen and (C₁-C₃)-alkoxy, preferably from hydrogen and methoxy; and,
R⁴ is halogen, preferably bromine, chloride, iodide, more preferably bromine,
or,
R¹ is alkoxy, optionally substituted, preferably selected from the group consisting of methoxy and a group -O-(CH₂)ₙ-R¹⁰ wherein n is 1, 2, 3 or 4 and R¹⁰ is selected from the group consisting of hydroxyl, -NMe₂, -OCONMe₂, -OCONH₂ and morpholine;
R² is hydrogen; and,
R⁴ is selected from the group consisting of hydrogen and alkoxy,
or,
R¹ is substituted alkoxy, preferably a group -O-(CH₂)ₙ-R¹⁰ wherein n is 1, 2, 3 or 4 and R¹⁰ is selected from the group consisting of hydroxyl, -NMe₂, -OCONMe₂, - OCONH₂ and morpholine;
R² is (C₁-C₃)-alkoxy, preferably methoxy; and
R⁴ is selected from the group consisting of hydrogen and alkoxy,
or any pharmaceutically acceptable salt thereof.

3. The rocaglaol derivative according to claim 2, wherein
R¹ is selected from the group consisting of methoxy or O-(CH₂)ₙ-OH wherein n is 1, 2 or 3, preferably a methoxy;
R² is hydrogen;
R³ is selected from the group consisting of -COOR⁵ and -CONH₂, wherein
R⁵ is methyl; and
R⁴ is halogen or methoxy, preferably bromine or methoxy.

4. The rocaglaol derivative according to claim 2, wherein
R¹ is substituted alkoxy selected from O-(CH₂)ₙ-R¹⁰ wherein n is 1, 2, 3 or 4 and R¹⁰ is selected from the group consisting of hydroxyl, -NMe₂, -OCONMe₂, -OCONH₂ and
morpholine, preferably R¹⁰ is hydroxyl;
R² is hydrogen or methoxy, preferably hydrogen;
R³ is selected from the group consisting of -COOR⁵ and -CONH₂, wherein
R⁵ is (C₁-C₃)-alkyl, preferably methyl; and
R⁴ is halogen or methoxy, preferably bromine or methoxy.

5. The rocaglaol derivative according to claim 2, wherein
R¹ is methoxy;
R² is selected from the group consisting of methoxy or hydrogen;
R³ is selected from the group consisting of -COOR⁵, and -CONH₂, wherein
R⁵ is methyl; and
R⁴ is halogen, preferably bromine.

6. The rocaglaol derivative according to claim 2, wherein
R¹ is methoxy;
R² is hydrogen;
R³ is -COOR⁵, wherein R⁵ is methyl; and
R⁴ is halogen or methoxy, preferably bromine or methoxy.

7. Rocaglaol derivative of the formula (I) 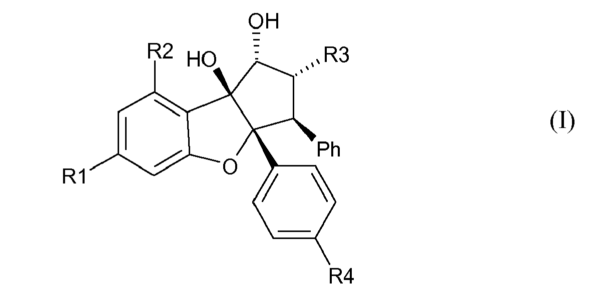 wherein
R³ is selected from the group consisting of hydrogen, -COOR⁵ and -CONR⁶R⁷, wherein
R⁵ is (C₁-C₃)-alkyl, preferably methyl, and
R⁶ and R⁷ are independently selected from the group consisting of hydrogen and (C₁-C₃)-alkyl, preferably from hydrogen and methyl;
and wherein
R¹ is methoxy;
R² is selected from the group consisting of hydrogen and (C₁-C₃)-alkoxy, preferably from hydrogen and methoxy; and,
R⁴ is bromine,
or any pharmaceutically acceptable salt thereof.

8. The rocaglaol derivative according to claim 7, wherein R¹ and R² are methoxy, R³ is hydrogen and R⁴ is bromine.

9. The rocaglaol derivative according to claim 7, wherein
R¹ is methoxy;
R² is hydrogen;
R³ is selected from the group consisting of hydrogen and -CON(CH₃)₂; and
R⁴ is bromine.

10. The rocaglaol derivative according to anyone of claims 2 to 9 as a medicament.

11. A pharmaceutical composition comprising a rocaglaol derivative according to anyone of claims 2 to 9 and a pharmaceutically acceptable carrier and/or excipient.

12. A pharmaceutical composition comprising a rocaglaol derivative according to anyone of claims 2 to 9 and an antineoplastic agent, preferably an anthracycline.

13. Product containing a rocaglaol derivative according to anyone of claims 2 to 9 and an antineoplastic agent, preferably an anthracycline, as a combined preparation for simultaneous, separate or sequential use in the treatment of cancer.

14. The rocaglaol derivative according to claim 1 or 2, wherein the rocaglaol derivative is a rocaglaol derivative according to anyone of claims 2 to 9.
